Europäisches Patentamt

European Patent Office

Office européen des brevets

(11) Publication number: **0 432 814 B1**

## EUROPEAN PATENT SPECIFICATION

(45) Date of publication of patent specification: **06.09.95**

(51) Int. Cl.[6]: **C07C 2/66**, C07C 15/085, //B01J29/70,C01B39/02

(21) Application number: **90202956.0**

(22) Date of filing: **08.11.90**

The file contains technical information submitted after the application was filed and not included in this specification

(54) **Process for alkylating benzene.**

(30) Priority: **16.11.89 IT 2241289**

(43) Date of publication of application:
**19.06.91 Bulletin 91/25**

(45) Publication of the grant of the patent:
**06.09.95 Bulletin 95/36**

(84) Designated Contracting States:
**AT BE CH DE DK ES FR GB GR LI LU NL SE**

(56) References cited:
EP-A- 0 030 084       EP-A- 0 055 046
EP-A- 0 272 830       US-A- 4 292 457
US-A- 4 871 444       US-A- 4 891 458

(73) Proprietor: **ENIRICERCHE S.p.A.**
**Corso Venezia 16**
**I-20121 Milan (IT)**

Proprietor: **ENICHEM SYNTHESIS S.p.A.**
**Via Ruggero Settimo 55**
**I-90139 Palermo (IT)**

Proprietor: **SNAMPROGETTI S.p.A.**
**Corso Venezia 16**
**I-20121 Milan (IT)**

(72) Inventor: **Cavani, Fabrizio**
**Via San Giovanni Bosco 78**
**I-41100 Modena (IT)**
Inventor: **Arrigoni, Virginio**
**Via Alfonso Cossa 2**
**I-20138 Milan (IT)**
Inventor: **Ghezzi, Roberto**
**Via Camelie 11**
**I-20095 Cusano Milanino**
**Milan (IT)**
Inventor: **Bellussi, Giuseppe**
**Via Alberto Scoto 44**
**I-29100 Piacenza (IT)**

(74) Representative: **Fusina, Gerolamo et al**
**Ing. Barzanò & Zanardo Milano S.p.A,**
**Via Borgonuovo, 10**
**I-20121 Milano (IT)**

**Description**

The present invention relates to a process for alkylating benzene with ethylene or propylene.

Said process is carried out in the presence of Beta zeolite as the catalyst, which zeolite is used as such, or modified by means of the isomorphous replacement of aluminum by boron, gallium or iron.

In particular, an object of the instant invention is the process for preparing cumene by alkylating benzene with propylene.

Cumene, or isopropylbenzene, is used above all for the production of phenol and acetone. At present, the most widely used synthetic route to obtain it is the condensation of benzene with propylene, catalysed by phosphoric acid supported on fossil meal. Although the cost of such a catalyst is not high, and it causes only small amounts of polyalkylation products to be formed as byproducts, such a catalyst is corrosive and therefore causes considerable problems to arise as regards the corrosion of the facilities used in order to carry out the process; furthermore, during the process, to the feedstock an exactly metered water amount has to be added in order to obtain a high reactivity of the catalyst. Moreover, such high reactivity values should be preserved unchanged over time. A further drawback is that such a catalyst cannot be regenerated and therefore causes disposal problems.

Another synthetic route used at the commercial level resorts to a slurry containing aluminum trichloride and hydrochloric acid as the condensation catalyst. This process is affected by problems of disposal and of corrosion, which are analogous to the above mentioned problems; furthermore, the catalyst is difficult to be separated from the resulting mixture of products resulting from the reaction, with the purity of such products being lower than of the products which can be obtained when phosphoric acid is used as the catalyst.

In order to obviate these drawbacks, condensation processes have been proposed in the past, in which the catalyst is a zeolite.

For example, the use of zeolites of ZSM-5 type is disclosed, such as in US-A-4,292,457, wherein a boralite having a structure of ZSM-5 type is said to be capable of catalysing the alkylation of benzene with propylene. However, such a type of zeolitic system, maybe owing to too small sizes of its internal channels, makes it possible for cumene to be only obtained with rather low selectivities.

On the other hand, a large-pore zeolite, such as ZSM-12 shows a good selectivity to cumene, but a low activity, and therefore high reaction temperatures should be used. Unfortunately, under these conditions, also undesired reactions are favoured, such as, e.g., cumene cracking, which can cause the catalyst to be rapidly deactivated.

Furthermore, a large number of patents exist which disclose the use, for cumene synthesis, of zeolites of Faujasite type, suitably modified by means of special treatments, such as the stabilization by steam treatment, or by means of treatments of exchange with rare earths.

In particular, zeolite of Y type is the most widely used one. It is characterized by a good activity at temperatures comprised within the range of from 130°C to 180°C and shows a good selectivity to the desired product. However, such a selectivity shows rapidly decreasing values with increasing values of benzene conversion, and therefore one has to operate with rather high molar ratios of benzene to propylene in the feedstock to the process, in order to limit the reactions of polyalkylation. Of course, such high ratios cause considerable costs for benzene recycle.

Also Beta zeolites have been used as catalysts for the alkylation of aromatic substrates with olefins. In particular, US-A-4,871,444 discloses a process for alkylating 1-methylnaphthalene with 1-butene in presence of beta zeolites. It can be observed, however, that values of conversion after one hour are about 5%. At this conversion value, corresponds a selectivity of 90% when the used catalyst is a zeolite beta with binder, while no selectivity is calculable when a zeolite beta without binder is used as catalyst.

EP-A-030084 discloses the use of beta zeolites in the alkylation of benzene with 1-dodecene, but the values of olefin conversion is 38% and the value of selectivity to phenyl-dodecane is 47%.

EP-A-272830 discloses, inter alia, a process for alkylating benzene with ethylene or propylene.

It has now been found that the beta zeolite is able to catalyze the alkylation of benzene with ethylene or propylene with very good results, with respect to both the conversion and the selectivity.

The present invention therefore provides a process for alkylating benzene with ethylene or propylene, said process comprising the step of bringing benzene into contact with ethylene or propylene in the presence of Beta zeolite as the catalyst, in its form in which H+ ion has partially or totally replaced the contained metal cation, with said Beta zeolite being used in its pristine state or modified by means of the isomorphous substitution of aluminum by boron, gallium or iron.

In particular, a preferred object of the present invention is a process for preparing cumene by alkylation of benzene with propylene, in the presence of the above disclosed catalyst.

This catalytic system displays a high activity and a high selectivity to cumene. Such a selectivity is independent from benzene conversion, i.e., by operating according to the process of the present invention the reaction of polyalkylation of benzene is minimized and therefore operating with an extremely large excess of this reactant -- as required by the processes known from the prior art -- is no longer necessary.

The process can be carried out in the gas phase, or in liquid or mixed phase, and batchwise, or in semi-continuous or continuous mode. The reaction temperature is selected from the range of from 100°C to 300°C, and preferably of from 110°C to 200°C; the pressure is comprised within the range of from 1.013 MPa to 5.065 MPa (10 to 50 atm), preferably of from 2.533 MPa to 3.546 MPa (25 to 35 atm), and the global hourly space velocity WHSV at which the reactants are fed is selected from within the range of from 0.1 to 200 hours$^{-1}$ and preferably of from 1 to 10 hours$^{-1}$.

Beta zeolite, disclosed in US-A-3,308,069 is a porous crystalline synthetic material having the following composition:

$$[(x/n)M (1 + 0.1\text{-}x)TEA] AlO_2 . ySiO_2 . wH_2O$$

wheren x is smaller than 1, y is comprised within the range of from 5 to 100, w is comprised within the range of from 0 to 4, M is a metal belonging to the groups IA, IIA, IIIA, or is a transition metal and TEA is tetraethyl-ammonium.

Modified forms of Beta zeolite can be obtained by means of the partial or total isomorphous substitution of aluminum by boron. For example, in BE-A-877,205 a porous cristalline borosilicate is disclosed, which is given the name of Boralite B. In EP-A-55,046 a zeolite isomorphous with Beta zeolite is disclosed, in which aluminum is partially replaced by boron, iron or gallium.

The catalysts are used in the acid form, i.e., in their form in which H$^+$ ion has partially or totally replaced the initially contained metal cation. This substitution is accomplished, according to the prior art, by means of a preliminary exchange with ammonium ion and subsequent calcination.

The catalysts can be used in mixture with suitable binding agents, such as, e.g., silicon, aluminum, zirconium, magnesium oxides, or natural clays, or combinations thereof.

The zeolite and the binding agent are mixed in relative amounts comprised within the range of from 50:50 to 95:5, preferably of from 70:30 to 90:10. The mixture of the two components is then compacted into the end desired shape for the catalyst, e.g., as cylindrical extrudates.

In the process for cumene preparation, the value of the molar ratio of benzene to propylene in the feedstock to the reaction can be comprised within the range of from 2 to 30 and is preferably comprised within the range of from 4 to 15.

The reactants can be fed to the reactor both from down upwards, and vice-versa. The heat developed during the process can be controlled by means of the injection of inert paraffins at various levels of the catalytic bed.

The regeneration of the catalyst is obtained by thermal treatment in air, e.g. at a temperature comprised within the range of from 500°C to 800°C. The useful life time between two successive regenerations is very long: e.g., an alumina-bound Beta zeolite does not show any decrease in its catalytic power after 200 hours of use in the alkylation of benzene with propylene carried out at 150°C and under a pressure of 3.039 MPa (30 atm).

In the following examples are reported, which illustrate the preparation of cumene and ethylbenzene with the use, as the catalyst, of Beta zeolite in its pristine state, or of Beta zeolite in which during the synthesis aluminum atoms have been partially replaced by boron atoms. Such examples carried out according to the method of the present invention are compared to the results which can be obtained when the zeolitic systems known from the prior art are used. From this comparison it emerges, in particular, that the process of the present invention displays a large number of advantages over the process known from the prior art in which Zeolite Y is used as catalyst, which process has been heretofore the one which made it possible the best results to be obtained.

Such advantages consist in a higher activity and higher selectivity, with the values of selectivity being independent from benzene conversion values.

Example 1

Preparation of Beta-zeolite

58.8 g of tetraethylammonium hydroxide at 40% by weight in aqueous solution and 1.9 g of sodium aluminate are added to 58.4 g of demineralized water. The resulting mixture is heated up to approximately

3

80°C and is kept stirred until complete dissolution is achieved. The so obtained clear solution is added to 37.5 g of colloidal silica Ludox HS at 40% by weight. A homogeneous suspension with a pH value of 14 is obtained, and is charged to a steel autoclave inside which said suspension is allowed to crystallize under hydrothermal conditions, at 150°C for 10 days, under static conditions and under its autogenous pressure. The crystallized product is filtered off, is washed, is dried 1 hour at 120°C, is calcined for 5 hours at 550° and is exchanged into its acid form by treatment with ammonium acetate and subsequent calcination.

At the chemical analysis, the so obtained sample shows the following composition, expressed as the molar ratio:

$$SiO_2/Al_2O_3 = 19.3$$

The product was characterized by X-ray powder diffraction.

Example 2

Preparation of B-Al-BOR-B zeolite

A first solution is prepared by adding 0.9 g of $Al(NO_3)_3.9H_2O$ previously dissolved in 11 g of demineralized water to 42 g of tetraethylammonium hydroxide at 40% by weight in aqueous solution.

A second solution is prepared by dissolving 4.6 g of NaOH and 9.4 g of boric acid in 33 g of demineralized water. The mixture is heated up to about 80°C and is kept stirred at this temperature until boric acid is completely dissolved. The first solution is then added to the second solution and the resulting mixture is added to 76 g of colloidal silica Ludox AS at 30% by weight. The so obtained solution, having a pH value = 12.2, is placed to crystallize under hydrothermal and static conditions, at 150°C for 7 days, under its autogenous pressure.

The end product which is obtained is filtered off, is washed by being re-dispersed in water, is dried at 120°C for 1 hour, is calcined at 550°C for 5 hours and is exchanged into its acid form.

At the chemical analysis the so obtained sample, characterized by X-ray diffraction analysis (XRD) shows the following composition:

$$SiO_2/B_2O_3 = 80$$
$$SiO_2/Al_2O_3 = 225$$

Examples 3-7

The zeolite Beta prepared according to Example 1, in powder form, as catalyst, is charged to an autoclave of 0.5 l of capacity, equipped with magnetic stirring and electric heating means. The system is evacuated and then 100 cc of benzene and subsequently 6 g of propylene are charged to it (the molar ratio of benzene/propylene in the mixture of reactants fed to the reaction is = 7.4; the maximum benzene conversion obtained with a stoichiometry of 1:1 is of 13.3%). The reaction temperature is rapidly increased up to 150°C and the autoclave is then pressurized to 3.039 MPa (30 atm) with nitrogen.

In the following Table the results are reported, which are obtained with variable reaction times (t), expressed as hours, and with variable amounts of charged catalyst (q), expressed as grams. The results are reported as the molar selectivity of benzene converted into cumene (indicated by the symbol "C9/C6 Sel."), as a function of the molar conversion of same benzene (indicated by the symbol "C6 Con."):

| Test N. | g/t | C6 Con. % | C9/C6 Sel. % |
|---|---|---|---|
| 3 | 0.1/0.5 | 1.7 | 96.5 |
| 4 | 0.1/1 | 5.2 | 96.4 |
| 5 | 0.2/1 | 7.9 | 96.3 |
| 6 | 0.5/1 | 11.0 | 95.9 |
| 7 | 1.0/1 | 12.0 | 96.1 |

From this data, both the high activity of Beta zeolite and the high selectivity thereof clearly appear. In particular, the present Applicant wishes to underline that, contrarily to what occurs for the catalytic systems known from the prior art, such a selectivity does not vary with increasing values of benzene conversion.

4

Examples 8-11

The process is carried out as in the preceding case, with B-Al-BOR-B zeolite prepared according to Example 2 being used as the catalyst.

The results are reported in the following Table:

| Test N. | g/t | C6 Con. % | C9/C6 Sel. % |
|---|---|---|---|
| 8 | 0.1/1 | 2.2 | 96.4 |
| 9 | 0.2/1 | 5.6 | 96.5 |
| 10 | 0.4/1 | 6.6 | 96.7 |
| 11 | 0.5/1 | 11.3 | 95.3 |

The data reported in the above Table evidence that Beta zeolite and B-Al-BOR-B zeolite display very similar values of activity and practically identical values of selectivity to cumene, with benzene conversion being the same.

Examples 12-16 (Comparative Examples)

The synthesis of cumene was carried out under the same conditions as of the preceding Example, with a Zeolite Y by Union Carbide marketed under the trade name LZ-Y 72 being used as the catalyst.

The results obtained are summarized in the following Table:

| Test N. | g/t | C6 Con. % | C9/C6 Sel. % |
|---|---|---|---|
| 12 | 0.3/1 | 2.8 | 89.3 |
| 13 | 0.5/1 | 4.2 | 89.5 |
| 14 | 1/1 | 6.4 | 88.2 |
| 15 | 2/1 | 8.4 | 86.7 |
| 16 | 4/1 | 9.6 | 86.1 |

These data, compared to the data obtained from Examples 3-11, set forth that not only zeolite Y results to be less active than pristine Beta zeolite and Beta zeolite modified by means of the partial isomorphous substitution with boron, but also results to be less selective than them. In particular, not only the initial selectivity results to be lower, i.e. the selectivity extrapolated to no benzene conversion, but also the decrease in selectivity with increasing values of such conversion is more pronounced.

Examples 17-19 (Comparative Examples)

The synthesis of cumene was carried out under the same conditions as of the preceding Examples, with a Y zeolite marketed by Toyo Soda under the trade name TSZ-HUA 330 being used as the catalyst.

The results obtained are summarized in the following Table:

| Test N. | g/t | C6 Con. % | C9/C6 Sel. % |
|---|---|---|---|
| 17 | 0.1/1 | 3.2 | 92.0 |
| 18 | 0.2/1 | 8.1 | 89.1 |
| 19 | 0.3/1 | 9.4 | 87.5 |

Whilst the activity of this catalytic system is comparable to the activity of Beta zeolite, the selectivity thereof is considerably lower.

Example 20 (Comparative Example)

Preparation of B-Al-ZSM-5

62 g of Al(NO$_3$)$_3$.9H$_2$O dissolved in 430 g of tetrapropylammonium hydroxide at 31.25% by weight and 155 g of boric acid are added to 4 l of demineralized water. The resulting mixture is heated up to approximately 80°C and is kept stirred until the complete dissolution of boric acid is achieved, then 2580 g of tetraethyl orthosilicate is added and the resulting mixture is kept with stirring at this temperature until hydrolysis is complete. The reaction mixture is then diluted with 800 g of demineralized water and the so obtained clear solution, having a pH value of 11.3, is charged to a steel autoclave in order to ba caused to crystallize under hydrothermal conditions, with stirring, at 180°C for 15 hours and under its autogenous pressure. The end product is centrifuged off, is washed by being redispersed in water, is dried 1 hour at 120°C, is calcined for 5 hours at 550°C and is exchanged into its acid form.

At the chemical analysis, the so obtained sample, characterized by analysis at X-rays, at the chemical analysis shows the following molar composition:

$$SiO_2/B_2O_3 \ = \ 144.76$$
$$SiO_2/Al_2O_3 \ = \ 148.44$$

Example 21 (Comparative Example)

Preparation of B-Al-ZSM-12

7.8 g of NaOH and 2.7 g of H$_3$BO$_3$ are dissolved in 54.9 g of triethylammonium hydroxide in aqueous solution at 15.9% by weight. Then, 41 g of demineralized water is added, in which 1.2 g of Al(NO$_3$)$_3$.9H$_2$O was previously dissolved. The so obtained clear solution is added to 43.6 g of Ludox AS at 30% by weight. A homogeneous white suspension with a pH value of 12.3 is obtained, and is charged to a steel autoclave in which it is allowed to crystallize under hydrothermal conditions, at 175°C for 5 days, under static conditions and under its autogenous pressure.

The so obtained product is filtered off, is washed, is dried 1 hour at 120°C, is calcined for 5 hours at 550°C and is exchanged into its acid form.

The so obtained sample was characterized by X-ray spectroscopy, and at the chemical analysis it shows the following molar composition:

$$SiO_2/B_2O_3 \ = \ 113.5$$
$$SiO_2/Al_2O_3 \ = \ 124.4$$

Examples 22-26 (Comparative Examples)

The synthesis of cumene was carried out under the same conditions as of Example 3-7, by using a ZSM-5 zeolite (synthesized according to Example 8 of GB-A-1,402,981), B-Al-ZSM-5 zeolite (synthesized according to Example 20 of the present application), B-Al-ZSM-12 zeolite (synthesized according to Example 21 of the present application), all in their protonic form and as powders.

The results obtained are summarized in the following Table:

| Test N. | Catalyst | g/t | C6 Con. % | C9/C6 Sel. % |
|---------|-------------|-----|-----------|--------------|
| 22 | ZSM-5 | 1/1 | 0.4 | 90.8 |
| 23 | B-Al-ZSM-5 | 1/1 | 0.6 | 95.0 |
| 24 | B-Al-ZSM-12 | 1/1 | 2.0 | 97.7 |
| 25 | B-Al-ZSM-12 | 1/3 | 4.0 | 97.5 |
| 26 | B-Al-ZSM-12 | 2/3 | 9.3 | 97.0 |

All these zeolitic systems, known from the prior art, result to be selective to cumene, but poor as regards benzene conversion.

6

Examples 27-29

Some cumene preparations were carried out under the same conditions as of Examples 3-7, using an amount of 11.4 g of propylene, corresponding to a molar ratio of benzene/propylene in the reactant mixture charged to the reaction, of 4.2, and to a maximum theoretical conversion of benzene of 24%, on considering a stoichiometry of 1:1.

The results obtained are reported in the following Table, in which the values of molar selectivity to diisopropylbenzenes relatively to converted benzene (C12/C6 Sel.) are additionally reported:

| Test N. | g/t | C6 Con. % | C9/C6 Sel. % | C12/C6 Sel. % |
|---------|------|-----------|--------------|---------------|
| 27 | 0.2/1 | 3.9 | 94.9 | 4.6 |
| 28 | 0.5/1 | 7.6 | 94.1 | 5.3 |
| 29 | 1/1 | 17.3 | 92.1 | 7.0 |

These data confirm that by operating according to the process of the present invention, a considerably high selectivity is obtained at both high and low values of benzene conversion. The byproducts which are formed are diisopropylbenzenes, whilst different alkylates, or propylene oligomers are practically absent. Therefore, differently from as known heretofore from the prior art, it is possible now to operate with very low molar ratios in the feedstock charged to the reaction as well, with the productivity to cumene being increased and the high values of selectivity being retained.

Example 30

1 g of Beta zeolite powder prepared according to Example 1 is charged to an autoclave of 0.5 l of capacity, equipped with a magnetic-drive stirrer and electrical heating means.

The system is evacuated and 200 ml of benzene is charged to it; thereafter, 6.5 g of ethylene is charged. The molar ratio of benzene/ethylene in the mixture charged to the reaction is of 9.7; the highest conversion which can be obtained for benzene, on considering a 1:1 stoichiometry, is 10.3%.

The temperature of the autoclave is rapidly increased up to 180°C and the autoclave is then pressurized with 4.052 MPa (40 absolute atm) with nitrogen.

After a 4-hour reaction, benzene conversion is of 6.8% and the molar selectivity to ethylbenzene is of 97.2%.

Example 31

Under the same conditions as of the preceding Example, 1 g of zeolite B-Al-BOR-B prepared according to Example 2 is used as the catalyst.

After a 3-hour reaction time, the conversion of benzene is of 3.0% and the molar selectivity to ethylbenzene is of 97.4%.

Examples 32-33

1 g of Beta zeolite powder, prepared according to Example 1, is charged to an autoclave of 0.5 l of capacity equipped with magnetic-drive stirring means and electrical heating means.

The system is evacuated and 200 ml of benzene is charged to it; then, 12.1 g of ethylene is charged. The molar ratio of benzene/ethylene in the reactants charged to the reaction is of 5.2; the maximum conversion obtainable for benzene, on considering a 1:1 stoichiometry, is of 19.2%.

The temperature of the autoclave is rapidly increased up to 180°C and the autoclave is then pressurized to 4.052 MPa (40 abs.atm) with nitrogen.

In the following Table, the results obtained from tests at different reaction times ("t" column, hours) are reported. The data relate to the conversion of benzene (C6 Con.) and to the molar selectivity of ethylbenzene, referred to the same benzene (C8/C6 Sel.):

EP 0 432 814 B1

| Test N. | t | C6 Con. % | C8/C6 Sel. % |
|---|---|---|---|
| 32 | 1 | 11.9 | 95.6 |
| 33 | 5 | 14.9 | 94.5 |

Examples 34-36

The tests are carried out in the same way as of Examples 32-33, with the test runs being carried out at 200°C and using 2 g of B-Al-BOR-B zeolite prepared according to Example 2 as the catalyst.
The results are reported in the following Table:

| Test N. | t | C6 Con. % | C8/C6 Sel. % |
|---|---|---|---|
| 34 | 1 | 4.6 | 96.7 |
| 35 | 2 | 9.0 | 95.3 |
| 36 | 4 | 11.7 | 93.8 |

**Claims**

1. Process for alkylating benzene comprising the step of bringing said compound into contact with ethylene or propylene in the presence of Beta zeolite as the catalyst, in its form in which H + ion has partially or totally replaced the contained metal cation, with said Beta zeolite being used in its pristine state or modified by means of the isomorphous substitution of aluminum by boron, gallium or iron.

2. Process according to claim 1 for preparing cumene, characterized in that benzene is brought into contact with propylene.

3. Process according to claim 1, characterized in that the temperature range within which the alkylation is carried out is of from 100°C to 300°C.

4. Process according to claim 1, characterized in that the pressure range within which the alkylation is carried out is of from 1.013 MPa (10 atm) to 5.065 MPa (50 atm).

5. Process according to claim 1, characterized in that the molar ratio of benzene to ethylene or propylene is comprised within the range of from 2 to 30.

6. Process according to claim 1, characterized in that the global hourly space velocity WHSV of feed of the reactants consisting of benzene and ethylene or propylene is comprised within the range of from 0.1 to 200 hours$^{-1}$.

7. Process according to claim 3, characterized in that the temperature is comprised within the range of from 110°C to 200°C.

8. Process according to claim 4, characterized in that the pressure is comprised within the range of from 2.533 MPa (25 atm) to 3.546 MPa (35 atm).

9. Process according to claim 5, characterized in that the molar ratio of benzene to ethylene or propylene is comprised within the range of from 4 to 15.

10. Process according to claim 6, characterized in that the global hourly space velocity is comprised within the range of from 1 to 10 hours$^{-1}$.

11. Process according to claim 1, characterized in that it is carried out in gas-phase, in the liquid phase or in a mixed-phase system.

8

**12.** Process according to claim 1, characterized in that the catalyst is mixed with one or more binding agents selected from silicon oxide, aluminum oxide, zirconium oxide, magnesium oxide or natural clays.

**Patentansprüche**

**1.** Verfahren zum Alkylieren von Benzol, umfassend die Stufe des Inberührungbringens dieser Verbindung mit Ethylen oder Propylen in Gegenwart von β-Zeolith als Katalysator in dessen Form, in welcher das H⁺-Ion partiell oder vollständig das enthaltene Metallkation ersetzt hat, wobei dieser β-Zeolith in seiner ursprünglichen Form oder modifiziert durch die isomorphe Substitution von Aluminium durch Bor, Gallium oder Eisen verwendet wird.

**2.** Verfahren nach Anspruch 1 zur Herstellung von Cumol, dadurch gekennzeichnet, daß Benzol mit Propylen in Kontakt gebracht wird.

**3.** Verfahren nach Anspruch 1, dadurch gekennzeichnet, daß der Temperaturbereich, in welchem die Alkylierung ausgeführt wird, von 100°C bis 300°C beträgt.

**4.** Verfahren nach Anspruch 1, dadurch gekennzeichnet, daß der Druckbereich, in welchem die Alkylierung ausgeführt wird, von 1.013 MPa (10 Atmosphären) bis 5.065 MPa (50 Atmosphären) beträgt.

**5.** Verfahren nach Anspruch 1, dadurch gekennzeichnet, daß das Molverhältnis von Benzol zu Ethylen oder Propylen im Bereich von 2 bis 30 liegt.

**6.** Verfahren nach Anspruch 1, dadurch gekennzeichnet, daß die auf die Stunde bezogene Gesamtraumgeschwindigkeit WHSV des Einsatzmaterials aus den aus Benzol und Ethylen oder Propylen bestehenden Reaktanten im Bereich von 0,1 bis 200 h⁻¹ beträgt.

**7.** Verfahren nach Anspruch 3, dadurch gekennzeichnet, daß die Temperatur im Bereich von 110°C bis 200°C liegt.

**8.** Verfahren nach Anspruch 4, dadurch gekennzeichnet, daß der Druck im Bereich von 2.533 MPa (25 Atmosphären) bis 3.546 MPa (35 Atmosphären) liegt.

**9.** Verfahren nach Anspruch 5, dadurch gekennzeichnet, daß das Molverhältnis von Benzol zu Ethylen oder Propylen im Bereich von 4 bis 15 liegt.

**10.** Verfahren nach Anspruch 6, dadurch gekennzeichnet, daß die auf die Stunde bezogene Gesamtraumgeschwindigkeit im Bereich von 1 bis 10 h⁻¹ liegt.

**11.** Verfahren nach Anspruch 1, dadurch gekennzeichnet, daß es in der Gasphase, in der Flüssigphase oder in einem gemischtphasigen System ausgeführt wird.

**12.** Verfahren nach Anspruch 1, dadurch gekennzeichnet, daß der Katalysator mit einem oder mehreren, unter Siliziumoxid, Aluminiumoxid, Zirkonoxid, Magnesiumoxid oder natürlichen Tonen ausgewählten Bindemittel(n) vermischt ist.

**Revendications**

**1.** Procédé d'alkylation du benzène, qui comprend l'étape consistant à mettre ce composé en contact avec de l'éthylène ou du propylène, en présence d'une zéolithe bêta utilisée, en tant que catalyseur, sous une forme dans laquelle des ions H⁺ ont remplacé tout ou partie des cations métalliques qu'elle contenait, cette zéolithe bêta étant utilisée sous sa forme primitive ou sous forme modifiée par substitution isomorphe de l'aluminium par du bore, du gallium ou du fer.

**2.** Procédé de préparation du cumène, conforme à la revendication 1, caractérisé en ce que l'on met du benzène en contact avec du propylène.

3. Procédé conforme à la revendication 1, caractérisé en ce que l'on effectue l'alkylation à une température située dans l'intervalle allant de 100°C à 300°C.

4. Procédé conforme à la revendication 1, caractérisé en ce que l'on effectue l'alkylation sous une pression située dans l'intervalle allant de 1,013 MPa (10 atm) à 5,065 MPa (50 atm).

5. Procédé conforme à la revendication 1, caractérisé en ce que le rapport molaire du benzène à l'éthylène ou au propylène se situe dans l'intervalle allant de 2 à 30.

6. Procédé conforme à la revendication 1, caractérisé en ce que la vitesse spatiale horaire globale d'introduction des réactifs que sont le benzène et l'éthylène ou le propylène se situe dans l'intervalle allant de 0,1 à 200 $h^{-1}$.

7. Procédé conforme à la revendication 3, caractérisé en ce que la température se situe dans l'intervalle allant de 110°C à 200°C.

8. Procédé conforme à la revendication 4, caractérisé en ce que la pression se situe dans l'intervalle allant de 2,533 MPa (25 atm) à 3,546 MPa (35 atm).

9. Procédé conforme à la revendication 5, caractérisé en ce que le rapport molaire du benzène à l'éthylène ou au propylène se situe dans l'intervalle allant de 4 à 15.

10. Procédé conforme à la revendication 6, caractérisé en ce que la vitesse spatiale horaire globale se situe dans l'intervalle allant de 1 à 10 $h^{-1}$.

11. Procédé conforme à la revendication 1, caractérisé en ce qu'il est réalisé en phase gazeuse, en phase liquide ou dans un système à phase mixte.

12. Procédé conforme à la revendication 1, caractérisé en ce que le catalyseur est mélangé avec un ou plusieurs agents liants choisis choisis parmi de l'oxyde de silicium, de l'oxyde d'aluminium, de l'oxyde de zirconium, de l'oxyde de magnésium, et des argiles naturelles.